# EUROPEAN PATENT SPECIFICATION

(11) **EP 4 049 712 B1**
(45) Date of publication and mention of the grant of the patent: **31.12.2025**
(21) Application number: 22157788.5
(22) Date of filing: 21.02.2022
(51) Int. Cl.: A61N 1/05

(54) **SYSTEM FOR CONDUCTION SYSTEM PACING IMPLANTATION**
SYSTEM ZUR IMPLANTIERUNG VON HERZSCHRITTMACHERN
SYSTÈME D'IMPLANTATION DE STIMULATION DU SYSTÈME DE CONDUCTION

(30) Priority: 24.02.2021 US 202163152942 P; 28.01.2022 US 202217587275
(43) Date of publication of application: 31.08.2022
(73) Proprietor: Medtronic, Inc., Minneapolis, Minnesota 55432 (US)
(72) Inventor: Demmer, Wade M., Mounds View, 55112 (US); Haddad, Tarek D., Mounds View, 55112 (US)
(74) Representative: Zimmermann & Partner Patentanwälte mbB

(56) References cited:
- US-A1- 2002 077 669
- US-A1- 2008 188 918
- US-A1- 2015 042 646
- US-A1- 2015 294 082
- US-A1- 2017 246 461

## Description

### TECHNICAL FIELD

This disclosure is related to medical systems for the navigation of an apparatus within a heart.

### BACKGROUND

Implantable medical leads may be adapted to treat a wide variety of cardiac dysfunctions. Implantable medical leads include electrodes and/or other elements for physiological sensing and/or therapy delivery. Implantable medical leads allow the sensing/therapy elements to be positioned at one or more target locations for those functions. An electrode supported by the medical lead may establish electrical communication with tissues of the heart to deliver cardiac pacing using the native conduction system of the heart, such as the His bundle, right and left bundle branches and the Purkinje fibers. Mapping of the endocardial surface of the heart using the electrode or another component of the lead is often conducted to determine a satisfactory location for the electrode within the heart in order to provide adequate activation native conduction system. US 2008/188918 A1 discloses system for supporting implantation of cardiac pacemakers, and in particular for supporting implantation of biventricular cardiac pacemakers.

### SUMMARY

The claimed subject matter is definde in claim 1. All methods disclosed below are given for illustrative purposes and are not claimed as methods. In an example, a medical system comprises: an imaging device configured to generate an image representative of a subject heart of a patient and representative of an apparatus portion within the subject heart, wherein the apparatus portion is at least a portion of an apparatus, and wherein the apparatus portion includes an electrode; and processing circuitry configured to: receive image data representative of the image of a subject heart and the apparatus portion within the subject heart, acquire electrode position data indicative of a position of the electrode within the subject heart, and apply a machine learning algorithm to generate an output readable by a clinician indicating a likelihood of successful conduction system pacing based on the electrode position data, wherein the machine learning algorithm is trained using a training data set indicative of one or more positions of an implanted electrode within an anatomical heart when the implanted electrode successfully and unsuccessfully established conduction system pacing of the anatomical heart.

In an example, a medical system comprises: a medical device configured to establish electrical communication with a torso of a patient, wherein the medical device is configured to generate a signal representative of a physiological parameter of a subject heart of the patient; an apparatus including an apparatus portion supporting an electrode, wherein the apparatus is configured to generate an electrical signal when the electrode is in electrical communication with tissues of the subject heart, wherein the apparatus portion is configured to be steerable when the apparatus portion is within the subject heart; a visual display; an imaging device configured to generate an image of the subject heart and the apparatus portion within the subject heart; and processing circuitry configured to: receive image data representative of the image of the subject heart and the apparatus portion generated by the imaging device, extract features of the subject heart using the received image data and produce a representative image of the subject heart on the visual display using the extracted features; determine electrode position data indicative of a position of the electrode within the subject heart using the received image; receive the representative signal from the medical device; apply a machine learning algorithm to generate an output readable by a clinician indicating a likelihood of successful conduction system pacing based on at least one of the electrode position data or the signal from the medical device, wherein the machine learning algorithm is trained using a training data set indicative of one or more positions of an implanted electrode within an anatomical heart when the implanted electrode successfully and unsuccessfully established conduction system pacing of the anatomical heart.

In an example, a method comprises: positioning an apparatus having an apparatus portion including an electrode within a subject heart of a patient, wherein the apparatus is configured to generate an electrical signal when the electrode is in electrical communication with tissues of the subject heart, and wherein the apparatus portion is configured to be steerable when the apparatus portion is within the subject heart; generating an image of the subject heart and the apparatus portion using an imaging device; receiving, using processing circuitry operably coupled to the imaging device, image data representative of the image of the subject heart and the apparatus portion; acquiring, using the processing circuitry, electrode position data indicative of a position of the electrode within the subject heart, and generating, using the processing circuitry, an output readable by a clinician indicating a likelihood of successful conduction system pacing based on the electrode position data , wherein the processing circuitry generates the output using a machine learning algorithm trained using a training data set indicative of one or more positions of an implanted electrode within an anatomical heart when the implanted electrode successfully and unsuccessfully established conduction system pacing of the anatomical heart.

The details of one or more examples are set forth in the accompanying drawings and the description below. Other features, objects, and advantages will be apparent from the description and drawings, and from the claims.

### BRIEF DESCRIPTION OF DRAWINGS

FIG. 1 is a conceptual diagram illustrating an example lead implanted at an example target implant site.
FIG. 2 is a schematic illustration of an example medical system for navigation of a lead.
FIG. 3 is a flow diagram illustrating an example configuration of a medical system.
FIG. 4 is a functional block diagram of an external device.
FIG. 5 is a block diagram of a medical system including a network.
FIG. 6 illustrates an example technique for using a medical system to position an electrode.

### DETAILED DESCRIPTION

This disclosure describes a medical system including processing circuitry configured to operably couple to an imaging device configured to generate an image of at least a portion of an apparatus within a heart of a patient. The apparatus portion supports (e.g., mechanically supports) at least one electrode configured to penetrate cardiac tissue at or near a target site of the heart. The apparatus may be configured to navigate within the heart of a patient in order to establish conduction system pacing (CSP) of the heart using the electrode. The apparatus may include, for example, a lead configured to pass through vasculature of the patient such that at least some portion (e.g., a distal portion) of the lead resides within the heart, an implantable device configured to reside within the heart (e.g., reside within a chamber of the heart), a delivery catheter supporting the implantable device and configured to position the implantable device within the heart, and other such apparatuses configured to provide support (e.g., mechanical support) to the electrode.

The processing circuitry is configured to receive image data representative of the generated image from the imaging device and generate an output readable by a clinician indicating a likelihood of successful conduction system pacing based on electrode position data indicative of a position of the electrode within the heart. The processing circuitry is configured to determine the likelihood of success using a machine learning algorithm trained with a training data set indicative of successful and/or unsuccessful electrode placements within an anatomical heart. For example, the training data set may include data (e.g., imaging data) indicating electrode locations and resulting capture characteristics for past CSP procedures in order to train the machine learning algorithm to determine the likelihood of success based on the electrode position data. In some examples, the system may include a visual display and produce the output readable by the clinician on the visual display.

The imaging device may be configured to generate an image of a subject heart (e.g., the heart of a patient undergoing a procedure) using substantially non-invasive imaging methods, such as fluoroscopy, ultrasound, magnetic resonance imaging (MRI), computed tomography (CT), electro-anatomical mapping modalities (e.g., impedance mapping), or other methods. The processing circuitry may be configured to receive the image data from the imaging device and recognize (e.g., extract) physiological landmarks of the subject heart using the received image data. The processing circuitry may be configured to determine target sites on the subject heart using the recognized physiological landmarks. In examples, the processing circuitry is configured to acquire electrode position data indicative of a position of the electrode within the heart of the patient. The processing circuitry may be configured to acquire at least some portion of the electrode position data by generating at least some portion of the electrode position data using the received image data. The processing circuitry may be configured to acquire at least some portion of the electrode position data by receiving one or more signals (e.g., from the electrode, from an apparatus and/or apparatus portion supporting the electrode, and/or from a system configured to determine a position of the electrode within the heart of a patient) using the one or more signals. The processing circuitry is configured to generate the likelihood of success based on the electrode position data and the target sites of the subject heart identified. In some examples, the system provides a visual display indicating the electrode position data (e.g., a position of the electrode within the heart) and one or more target sites of the subject heart using the received image data from the imaging device.

For example, the imaging device may provide a fluoroscopic image of the subject heart and the lead within the subject heart to the processing circuitry. The processing circuitry may be configured to conduct landmark recognition of the received image data using an image recognition algorithm trained to identify physiological landmarks. For example, the image recognition algorithm may be configured to identify anatomical structures of the subject heart represented within the received image data, such as atria, ventricles, vena cava, anatomical valves, various regions of a human heart, and other physiological landmarks. The processing circuitry may be configured to determine one or more target sites on the subject heart using the identified physiological landmarks. The processing circuitry may be further configured to acquire electrode position data and ascertain a position of the electrode relative to the target sites. In examples, the medical system generates a output on the visual display indicating the ascertained position of the electrode relative to the target sites. The medical system may be configured to refresh the output, such that when the electrode position data indicates a position of the electrode within the subject heart has altered (e.g., been altered by a clinician), the medical system updates the visual display to indicate the new ascertained position of the electrode relative to the target sites. In examples, the processing circuitry (e.g., the image recognition algorithm) may configured to conduct landmark recognition of the subject heart using other image data of the subject heart (e.g., pre-recorded other image data) in combination with image data received from the imaging device, such as other image data obtained through fluoroscopy, electroanatomical mapping, computed tomography (CT) scans, magnetic resonance imaging (MRI), and/or other sources of other image data.

The processing circuitry of the medical system may be configured to use the electrode position data and the target sites in order to determine the likelihood of success for conduction system pacing of the subject heart with the electrode in the position indicated by the electrode position data. The likelihood of success may be based on one or more past procedures wherein an electrode was positioned within an anatomical heart for the generation of conduction system pacing. The processing circuitry includes a machine learning algorithm (e.g., an artificial intelligence (AI) algorithm) trained with a data set reflecting the past procedures. For example, the machine learning algorithm may be trained with a data set reflecting a plurality of relative positions between an electrode and various physiological landmarks of anatomical hearts in the past procedures, as well as an indicator of successful or unsuccessful conduction system pacing for the relative positions. The trained machine learning algorithm may be configured to subsequently receive data indicative of the present position of the electrode relative to the target sites determined for a subject heart (e.g., the heart of the patient undergoing a procedure) and indicate a likelihood of success for conduction system pacing with the electrode in the position indicated by the electrode position data, based on the training conducted using the training data set.

In examples, the processing circuitry is configured to utilize a first algorithm (e.g., an image recognition algorithm) to identify physiological landmarks of the subject heart and utilize a second algorithm to determine the likelihood of success for the electrode position data acquired using the physiological landmarks identified by the first algorithm. The processing circuitry may be configured to provide the image data of the subject heart received from the imaging device to the first algorithm. In examples, the first algorithm is an image recognition algorithm trained to identify anatomical structures of an anatomical heart (e.g., a human heart). The first algorithm may be configured to segment the received image data to identify the physiological landmarks. The processing circuitry may be configured to provide data representative of the physiological landmarks to the second algorithm. The second algorithm may be configured to use the representative data to map target sites for the subject heart. The processing circuitry (e.g., executing the second algorithm) may be configured to receive and/or interpret electrode position data indicative of a position of the electrode relative to the target sites, and provide the likelihood of success for conduction system pacing using the electrode position data. For example, the processing circuitry may receive and/or interpret the electrode position data to assess (e.g., determine) a present position and/or location of the electrode within the subject heart. In examples, the second algorithm is trained with the data set reflecting a plurality of electrode position datum and various physiological landmarks of anatomical hearts in the past procedures.

The medical system may include a medical device configured to establish electrical communication with a torso of the patient. The medical device may be configured to sense electrical and/or mechanical activity of the subject heart within the patient. In examples, the medical device includes a plurality of electrodes configured to establish electrical communication with the torso of the patient and generate one or more signals indicative of the electrical and/or mechanical activity of the subject heart. For example, the medical device may be an belt or vest (e.g., an ECG belt or vest) including the plurality of electrodes. In examples, the processing circuitry is configured to receive the one or more electrical signals indicative of the electrical and/or mechanical activity of the subject heart from the medical device. The processing circuitry may be configured to base the likelihood of success for given electrode position data based at least in part on the one or more indicative signals received from the medical device. In some examples, the medical device may be configured to provide the one or more indicative signals to the imaging device, and the imaging device may be configured to provide the image data of a subject heart using the one or more signals from the medical device. For example, the medical device may be configured to determine one or more pseudo-electric vectors (PEVs) representing electrical forces generated by the subject heart, and the imaging device may be configured to generate a representative image data of the subject heart using the one or more PEVs.

The electrode supported by the apparatus (e.g., supported by an apparatus portion) may be configured to establish electrical communication with the tissues of the subject heart (e.g., the heart of the patient undergoing a procedure). In examples, the processing circuitry is configured to receive an electrical signal from the electrode indicative of the electrical communication. The processing circuitry may be configured to base the likelihood of success for given electrode position data indicative of a position of the electrode in a subject heart based at least in part on the indicative signal received from the electrode.

The processing circuitry may be configured to evaluate the delivery of conduction system pacing to the subject heart by the electrode and provide feedback to the machine learning algorithm. For example, the processing circuitry may be configured to evaluate the delivery of conduction system pacing using at least one of the medical device (e.g., an ECG belt or vest) or the electrical signal from the electrode. The processing circuitry may be configured to provide a quality indicator reflective of the conduction system pacing delivered with the electrode in the position within the subject heart indicated by the electrode position data. The machine learning algorithm may be configured to engage in machine learning (e.g., supervised, unsupervised, and/or reinforcement learning) using the quality indicator. The quality indicator may be, for example, a vector of features constituting a description of the conduction system pacing delivered by the electrode. In examples, the processing circuitry is configured to receive one or more features indicative of the quality indicator from an input device configured to receive inputs from a clinician.

In examples, the medical system includes an external device configured to receive the image data of the subject heart and the apparatus portion and implement at least some portion of the machine learning algorithm. The external device may be, for example, any fixed or mobile computer system (e.g., a controller, a microcontroller, a personal computer, minicomputer, tablet computer, etc.) and may be generally described as including processing circuitry. In some examples, the external device is configured to receive the image data of the subject heart and the apparatus portion from the imaging device. The external device may include the visual display indicating a position of the electrode based on the electrode position data and one or more target sites determined using the machine learning algorithm. For example, the external device may be a tablet including an image device (e.g., a camera) configured to capture an image generated by the imaging device within a defined field-of-view and configured to implement the machine learning algorithm to produce the output readable by the clinician to indicate the likelihood of success. The external device may include the visual display (e.g., a graphical user interface) indicating the relative position between the electrode and the target sites determined for the subject heart. For example, the external device may be configured to receive a fluoroscopic image from the imaging device within the field-of-view of the image apparatus, generate image data representative of the fluoroscopic image, extract physiological landmarks of the subject heart using the image data and an image recognition algorithm, map one or more target sites on the subject heart using the extracted physiological landmarks, and provide a representative image of the subject heart and the apparatus portion illustrating the relative position between the electrode of the apparatus portion and the one or more target sites.

In examples, the medical system may be implemented using one or more computer programs implemented on programmable computers, such as computers that include, for example, processing capabilities, data storage (e.g., volatile or nonvolatile memory and/or storage elements), input devices, and output devices. Program code and/or logic described herein may be applied to input data to perform the functionality described herein and generate desired output information. The programs may be stored on any suitable device, e.g., a storage media, readable by a general or special purpose program running on a computer system (e.g., including processing apparatus) and configuring the computer system to perform functions described herein. Computer-implemented instructions, data structures, screen displays, and other data under aspects of the technology may be stored or distributed on computer-readable storage media, including magnetically or optically readable computer disks, as microcode on semiconductor memory, nanotechnology memory, organic or optical memory, or other portable and/or non-transitory data storage media. In some embodiments, aspects of the technology may be distributed over the Internet or over other networks (e.g., a Bluetooth network) on a propagated signal on a propagation medium (e.g., an electromagnetic wave(s), a sound wave) over a period of time, or may be provided on any analog or digital network (packet switched, circuit switched, or other scheme).

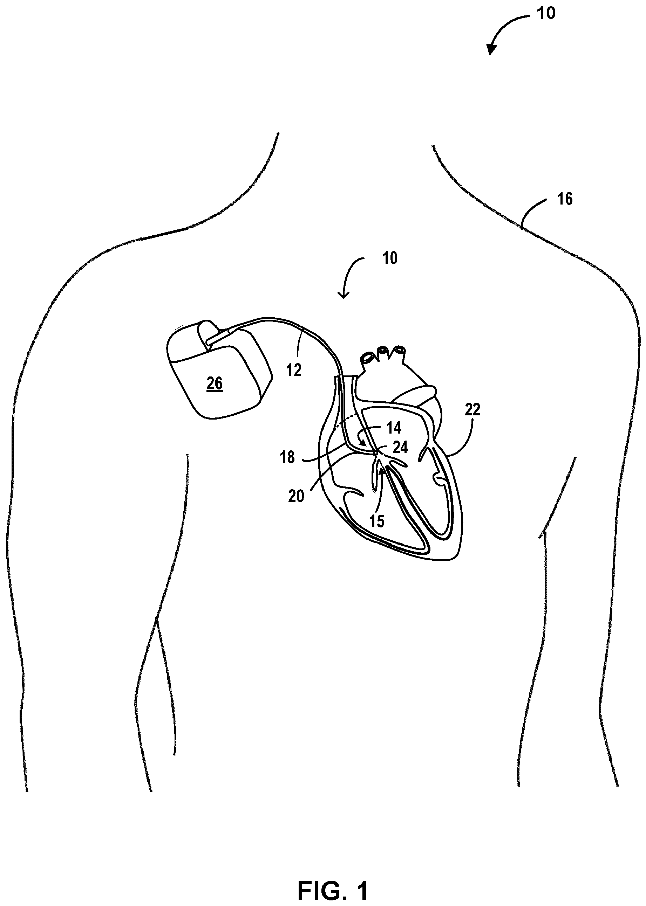

FIG. 1 is a conceptual diagram illustrating a portion of an example medical system 10 configured to deliver conduction system pacing (CSP) to a subject heart 22 of a patient 16. Medical system 10 includes an apparatus 12 supporting an electrode 24. In examples, apparatus 12 is an implantable medical lead extending from a device 26. In other examples, apparatus 12 may include an implantable device configured to reside within subject heart 22 (e.g., reside within a chamber of subject heart 22), a delivery catheter supporting an implantable device and configured to position the implantable device within subject heart 22, and/or other such apparatuses configured to provide support (e.g., mechanical support) to electrode 24. In some examples, apparatus 12 includes an elongated lead body 18 and/or an apparatus portion 20 generally positioned at a target site 14 within a patient 16. In examples, as illustrated in FIG. 1, target site 14 is a triangle of Koch region in the atrioventricular septal wall of the patient's heart. In examples, apparatus 12 may be oriented such that apparatus portion 20 positions at another portion of subject heart 22. For example, apparatus 12 may be oriented such that apparatus portion 20 generally positions at a target site 15 in the ventricular septal wall in the basal (e.g., high basal or high septal) region or apical (e.g., low septal or near the apex) region. Implantation in the triangle of Koch region of the atrioventricular septal wall may facilitate pacing of the His bundle or ventricular myocardium. Implantation in the basal region of the ventricular septal wall may facilitate pacing of one or both of the His bundle branches. Implantation in the apical region may facilitate pacing of Purkinje fibers. System 10 may include additional leads coupled to device 26 and extending into subject heart 22.

Electrode 24 may be configured to penetrate cardiac tissue at or near a target sites, such as target sites 14, 15. For example, electrode 24 of apparatus 12 may be configured to penetrate to a position at or near the left bundle branch (LBB), His bundle (HB), right bundle branch (RBB), other specialized conductive tissue, or other ventricular tissue of subject heart 22. In examples where electrode 24 is configured to penetrate to a position at or near the LBB or HB, electrode 24 may traverse the ventricular septum from right to left. In some examples, electrode 24 is configured to provide pacing to subject heart 22. Electrode 24 may be electrically connected to one or more conductors (not shown) extending through and/or within apparatus 12 from electrode 24. In some examples, the conductors are electrically connected to therapy delivery circuitry of device 26, with the therapy delivery circuitry configured to provide electrical signals through the conductor to electrode 24. Electrode 24 may conduct the electrical signals to the target tissue of subject heart 22, causing the cardiac muscle, e.g., of the ventricles, to depolarize and, in turn, contract at a regular interval. In examples, the cardiac pacing delivered via electrode 24 may be conduction system pacing (CSP) of subject heart 22, which may provide more physiologic activation and contraction of subject heart 22. Electrode 24 may also be connected to sensing circuitry of device 26 configured to sense activity of subject heart 22 via electrode 24. Electrode 24 may have various shapes such as tines, helices, screws, rings, and so on.

A clinician may maneuver at least some portion of apparatus 12 (e.g., apparatus portion 20) through the vasculature of patient 16 in order to position apparatus 12 at or near a target site, such as target site 14, 15. For example, the clinician may guide apparatus 12 through the superior vena cava (SVC), into the RA, and past the Tricuspid valve into the RV in order to access target site 14 on the atrioventricular septal wall. The clinician may guide apparatus 12 through the SVC to target site 15 on or in a ventricular septal wall of subject heart 22. In some examples, other pathways or techniques may be used to guide apparatus 12 into other target implant sites within the body of patient 16. Implantable medical system 10 may include a delivery catheter and/or outer member (not shown), and apparatus 12 may be guided and/or maneuvered within a lumen of the delivery catheter in order to approach target sites within subject heart 22. In some examples, apparatus 12 includes one or more tines (not shown) configured to grasp tissues at or near a target site (e.g., target sites 14, 15) and substantially secure a distal end of apparatus 12 to the target site. For example, the one or more tines may be configured to substantially secure the distal end of apparatus 12 to tissues of subject heart 22.

Conduct of conduction system pacing using an apparatus such as apparatus 12 is generally dependent on maneuvering apparatus 12 (e.g., apparatus portion 20) and electrode 24 to an adequate position within subject heart 22. In some examples, an initial site may be determined by a clinician observing an image of subject heart 22 using an imaging modality such as fluoroscopy. The clinician may conduct electrophysiological mapping of particular regions of subject heart 22 to locate an initial target site (e.g., target site 14, 15), then attempt to establish electrode 24 in electrical communication with the particular target site by observing subject heart 22 and apparatus 12 (e.g., apparatus portion 20) on the image. The adequacy of each potential position of electrode 24 may be evaluated through observation of the resulting capture characteristics (e.g., ECG and/or EGM signals) generated by subject heart 22. Further manipulation of apparatus 12 and electrode 24 may be required, depending on the resulting capture characteristics demonstrated. The procedural duration required to place electrode 24 at an adequate target site within subject heart 22 may be largely dependent on the imaging device employed and its ability to convey both the landmarks of subject heart 22 and the position of electrode 24 relative to the landmarks. In some examples, the relative positions between electrode 24 and a desired target site may be difficult to discern due to, for example, the need to navigate within a three-dimensional space using a two-dimensional image, physiological variations encountered in individual patients, difficulty in identifying the landmarks of subject heart 22 using the available image, or other reasons.

Disclosed herein is a medical system including an imaging device, an apparatus including an electrode, and processing circuitry operably coupled to the imaging device. The processing circuitry is configured to receive image data representative of a subject heart of a patient and generate an output readable by a clinician indicating a likelihood of successful conduction system pacing based on electrode position data indicative of a position of the electrode within the heart. The image data may be representative of the apparatus within the subject heart. The processing circuitry is configured to determine target sites for the electrode using recognized physiological landmarks of the subject heart determined from analysis of the received image data. The processing circuitry is configured to acquire electrode position data indicative of a position (e.g., a location) of the electrode within the subject heart and generate the likelihood of success for conduction system pacing based on the electrode position data and the target sites of the subject heart identified. The medical system may provide a visual display indicating an assessed position of the electrode and one or more target sites of the subject heart using the received image data from the imaging device. In examples, the medical system may provide an indication of the assessed position of the electrode and the one or more target sites of the subject heart using other image data of subject heart 22 (e.g., pre-recorded other image data) in combination with the received image data, such as other image data obtained through fluoroscopy, electroanatomical mapping, computed tomography (CT) scans, magnetic resonance imaging (MRI), and/or other sources of other image data.

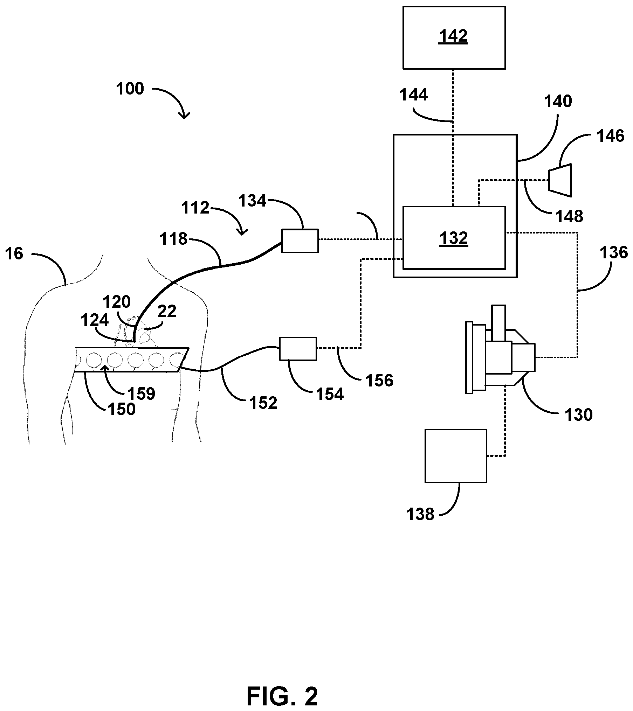

FIG. 2 illustrates is a conceptual diagram of a medical system 100 configured to identify and display potential target sites for the placement of electrodes configured to cause conduction system pacing of subject heart 22 within patient 16. Medical system 100 may include an imaging device 130, an apparatus 112, and processing circuitry 132. Apparatus 112 may include lead body 118, apparatus portion 120, and electrode 124, and may include apparatus circuitry 134. Processing circuitry 132 is operably coupled to imaging device 130 via communication link 136.

Imaging device 130 may be any type of imaging device configured to image, or provide images of, subject heart 22 of patient 16. Imaging device 130 may be configured to image at least a portion of apparatus 112 (e.g., apparatus portion 120) when apparatus portion 120 is positioned within subject heart 122. As used herein and below, apparatus portion 120 may refer to a portion of apparatus 112, where the portion is positioned within subject heart 122. For example, in some examples, apparatus portion 120 may be a distal portion of a lead and/or delivery catheter configured to position within subject heart 122 (e.g., in a chamber of heart 122) as another portion of the lead and/or delivery catheter resides outside of heart 122. In some examples, apparatus portion 120 may be a portion of an implantable device configured to reside in part or substantially wholly within subject heart 122 (e.g., reside at least in part or substantially wholly within a chamber of subject heart 122). Imaging device 130 may be configured to image subject heart 22 and/or apparatus portion 120 in a substantially non-invasive manner. Imaging device 130 may be configured to capture images of subject heart 22 and/or apparatus portion 120 using one or more image modalities, such as x-ray images (e.g., two dimensional x-ray images, three dimensional x-ray images, etc.), isocentric fluoroscopy, bi-plane fluoroscopy, ultrasound, computed tomography (CT), multi-slice computed tomography (MSCT), magnetic resonance imaging (MRI), high frequency ultrasound (HIFU), optical coherence tomography (OCT), intra-vascular ultrasound (IVUS), two dimensional (2D) ultrasound, three dimensional (3D) ultrasound, four dimensional ( 4D) ultrasound, intraoperative CT, intraoperative MRI, and/or others. In examples, imaging device 130 is configured to capture a plurality of consecutive images (e.g., continuously) to provide video frame data.

Imaging device 130 may include a display apparatus 138 configured to display information to a user, including graphical depictions of anatomy of subject heart 22, graphical depictions of locations of apparatus portion 120 and/or one or more electrodes such as electrode 124, graphical depictions of one or more target or candidate locations (e.g., target site 114, 115 (FIG. 1)), alphanumeric representations of one or more values, graphical depictions or actual images of implanted electrodes and/or leads, etc. For example, the display apparatus 138 may include a liquid crystal display, an organic light emitting diode screen, a touchscreen, a cathode ray tube display, or other devices configured to display information to a user.

Processing circuitry 132 is operably coupled to imaging device 130 via communication link 136 such that image data representative of the images captured by imaging device 130 may be transmitted to processing circuitry 132. Processing circuitry 132, in some examples, may include one or more processors that are configured to implement functionality and/or process instructions stored in a storage device. Processing circuitry 132 may include, for example, microprocessors, digital signal processors (DSPs), application specific integrated circuits (ASICs), field-programmable gate arrays (FPGAs), or equivalent discrete or integrated logic circuitry, or a combination of any of the foregoing devices or circuitry. Accordingly, processing circuitry 132 may include any suitable structure, whether in hardware, software, firmware, or any combination thereof, to perform the functions ascribed herein to processing circuitry 132.

In examples, processing circuitry 132 represents processing circuitry located within a an external device 140. In some examples, processing circuitry 132 may be entirely located within a housing of external device 140. In other examples, processing circuitry 132 may be located within external device 140 and another device or group of devices that are not illustrated in FIG. 2. As such, techniques and capabilities attributed herein to processing circuitry 132 may be attributed to any combination of external device 108 and other devices that are not illustrated in FIG. 2. External device 140 may be, for example, any fixed or mobile computer system (e.g., a controller, a microcontroller, a personal computer, minicomputer, tablet computer, etc.) and may be generally described as including substantially all or some portion of processing circuitry 132. In some examples, external device 140 is configured to receive and/or observe an image of subject heart 22 and apparatus portion 120 produced by imaging device 130 and generate image data representative of the image.

Processing circuitry 132 is configured to receive image data representative of the image of subject heart 22 and apparatus portion 120 of apparatus 112 from imaging device 130 and generate an output readable by a clinician indicating a likelihood of successful conduction system pacing based on electrode position data indicative of a position of electrode 124 within subject heart 22. Processing circuitry 132 is configured to determine the likelihood of success using a machine learning algorithm (e.g., an artificial intelligence (AI) algorithm) trained with a training data set indicative of successful electrode placements in conduction system pacing (CSP) procedures performed in the past on previous CSP patients. For example, the training data set may include a plurality of individual descriptions of individual past CSP procedures, with each individual description including individual electrode position data describing an individual position of an electrode within an anatomical heart and at least one success metric, with the success metric assessing the success of the conduction system pacing delivered to the anatomical heart with the electrode in the individual location. The individual descriptions of the training set may include, for example, a vector of features indicating the individual location of the electrode and the success metric. The success metric may include any assessment of the success of the individual past CSP procedure, including assessments determined and provided by clinicians performing the past CSP procedure, one or more resulting capture characteristics for the individual past CSP procedure, or some other assessment. The machine learning algorithm of processing circuitry 132 is configured to determine the likelihood of success for conduction system pacing based on the electrode position data indicative of the position of electrode 124 within subject heart 122, based on the training provided by the training data set.

Processing circuitry 132 may be configured to assess a position of electrode 124 within subject heart 22 using the electrode position data and base the indicated likelihood of success at least partially on the assessed position. In examples, processing circuitry 132 assesses the position of electrode 124 using the image data representative of the image of subject heart 22 and apparatus portion 120 generated by imaging device 130. In examples, processing circuitry 132 is configured to assess the position of electrode 124 relative to one or more physiological landmarks of subject heart 22. Processing circuitry 132 may be configured to conduct landmark recognition of subject heart 22 using an image recognition algorithm trained to identify the physiological landmarks of an anatomical heart. In examples, the image recognition algorithm is configured to use imaging data describing an anatomical heart to identify anatomical structures, such as atria, ventricles, vena cava, anatomical valves, various regions of a human heart, and other physiological landmarks. In examples, processing circuitry 132 is configured to assess the position of electrode 124 within subject heart 22 by determining one or more physiological landmarks of subject heart 22 using the received image data of heart 22, and assessing the position of electrode 124 within subject heart 22 by locating electrode 124 relative to one or more of the physiological landmarks. In examples, processing circuitry 132 (e.g., the image recognition algorithm) may configured to conduct landmark recognition of subject heart 22 using other image data of subject heart 22 (e.g., pre-recorded other image data) in combination with image data received from imaging device 130, such as other image data obtained through fluoroscopy, electroanatomical mapping, computed tomography (CT) scans, magnetic resonance imaging (MRI), and/or other sources of other image data.

In examples, apparatus portion 120 includes one or more physical structures such as radiopaque bands or other structures. Apparatus portion 120 may be configured such that the one or more physical structures have a substantially fixed spatial relationship to electrode 124. Processing circuitry 132 may be configured to recognize the one or more physical structures within the image data provided. In examples, processing circuitry 132 is configured to acquire electrode position data and/or assess the position of electrode 124 based at least in part on the recognition of the one or more physical structures and the substantially fixed spatial relationship to electrode 124.

In some examples, processing circuitry 132 is configured to receive an electrical signal from electrode 124 and acquire electrode position data and/or assess the position of electrode 124 using the electrical signal. For example, when electrode 124 is in electrical communication with tissues of subject heart 22, processing circuitry may be configured to receive the electrical signal and assess a location where electrode 124 is likely to be located within subject heart 22, based on the signal received. Processing circuitry 132 may be configured to assess the location based on a comparison of the received signal from electrode 124 with signals expected from various locations within the conduction system of subject heart 22. In examples, the processing circuitry 132 is configured to acquire electrode position data and/or assess the position of electrode 124 using an algorithm (e.g., the machine learning algorithm or another algorithm) trained with a training data set indicative of signals received when similar electrodes were implanted at various locations within the conduction system of anatomical hearts. In examples, the algorithm is trained to receive the electrical signal from electrode 124 as an element of an input vector and assess the location of electrode 124 within the conduction system of subject heart 22 using the electrical signal received from electrode 124.

In examples, processing circuitry 132 is configured to determine one or more target sites (e.g., target sites 114, 115 (FIG. 1)) on subject heart 22 using the physiological landmarks of heart 22 recognized with the image data provided by imaging device 130. Processing circuitry may be further configured to determine a relative location of electrode 124 compared to the determined target sites. In examples, medical system 100 includes a visual display 142 operably coupled to processing circuitry 132 (e.g., via communication link 144), and processing circuitry 132 is configured to generate a representative image of subject heart 22 on visual display 142 using the identified physiological landmarks. Processing circuitry 132 may include (e.g., superimpose) the determined target sites on the representative image. In examples, processing circuitry 132 is configured to further generate a representative image of apparatus portion 120 and/or electrode 124 on visual display 142, based on the position of electrode 124 within subject heart 22 indicated by the electrode position data. Processing circuitry 132 may be configured to include the representative image of apparatus portion 120 and/or electrode 124 within the representative image of heart 22, such that a clinician viewing visual display 142 may assess the location of electrode 124 relative to the target sites provided on the representative image of heart 22.

The representative image of subject heart 22, apparatus portion 120, and/or electrode 124 may be two-dimensional image or a three-dimensional image. In examples, processing circuitry 132 receives imaging data representative of a two-dimensional images from imaging device 130 and uses an image recognition algorithm to generate a multi-dimensional point cloud representative of subject heart 22, apparatus portion 120, and/or electrode 124 from the imaging data representative of the two-dimensional image. Processing circuitry 132 may be configured to generate the representative image of subject heart 22, apparatus portion 120, and/or electrode 124 on visual display 142 using the multi-dimensional point cloud. In examples, the multi-dimensional point cloud includes at least three dimensions and processing circuitry 132 is configured to generate a three-dimensional representative image of subject heart 22, apparatus portion 120, and/or electrode 124 using the multi-dimensional point cloud. Medical system 100 (e.g., processing circuitry 132) may be configured to periodically refresh the representative image generated on visual display 142, such that when the electrode position data indicating the position of electrode 124 within subject heart 22 alters (e.g., through the action of a clinician), medical system 100 updates the representative image of visual display 142 to indicate a new relative position of electrode 124 within the representative image of display 142. Processing circuitry 132 may be configured to indicate a likelihood of success for conduction system pacing with electrode 124 in the new relative position, based on the training conducted using the training data set.

In examples, instead of or in addition to communication link 136, medical system 100 includes an image device 146 configured to operably connect processing circuitry 132 and imaging device 130. Image device 146 may be, for example, a camera. Image device 146 may be configured to generate the image data representative of an image of heart 22, apparatus portion 120, and/or electrode 124 and provide the representative data to processing circuitry 132 (e.g., via communication link 148). For example, image device 146 may define a field-of-view and be configured to generate the image data based on the visual appearance of one or more objects within the field-of-view. Hence, medical system 100 may be configured such, when display apparatus 138 displays an image of subject heart 22, apparatus portion 120, and/or electrode 124, the image may be placed within the field-of-view of image device 146 to cause image device 146 to generate image data representative of the image displayed. Image device 146 may be configured to provide the resulting image data to processing circuitry 132.

For example, imaging device 130 may be set up to generate an image of subject heart 22, apparatus portion 120, and/or electrode 124 within patient 16 and display the image to a clinician on a video screen of display apparatus 138. The image displayed may be, for example, a two-dimensional image of subject heart 22, apparatus portion 120, and/or electrode 124 obtained using fluoroscopy or some other imaging method. External device 140 may be a portable device (e.g., a tablet) including image device 146 (e.g., a camera) defining a field-of-view and configured to be repositioned within a space (e.g., an operating room) by a clinician. Medical system 100 may be configured to allow external device 140 to be repositioned such that the video screen of display apparatus 138 is within the field-of-view of image device 146. External device 140 may be configured to capture an image of the video screen and generate image data representative of the image, and subsequently provide the image data to processing circuitry 132. Processing circuitry 132 may use the image data provide an output indicating a likelihood of success for conduction system pacing as disclosed herein.

In examples, processing circuitry 132 is configured to utilize a first algorithm to identify physiological landmarks of subject heart 22 and utilize a second algorithm to determine the likelihood of success for conduction system pacing based at least in part on the physiological landmarks identified by the first algorithm. Processing circuitry 312 may be configured to provide the image data to the first algorithm. The first algorithm may be an image recognition algorithm trained to identify anatomical structures of an anatomical heart such as subject heart 22. For example, the first algorithm may be trained to identify anatomical structures of subject heart 22 such as atria, ventricles, vena cava, anatomical valves, and other various regions of subject heart 22. Human heart, and other physiological landmarks. In examples, the first algorithm is configured to conduct image segmentation of the image of subject heart 22, apparatus portion 120, and/or electrode 124 using the image data provided by processing circuitry 132 (e.g., from image device 146 and/or imaging device 130). In some examples, the first algorithm is configured to segment the received image to identify physiological landmarks of subject heart 22. The first algorithm may be configured to receive imaging data representative of one or more two-dimensional images of subject heart 22 and generate representative image data describing a three-dimensional representation of subject heart 22. In examples, processing circuitry 132 (e.g., the first algorithm) is configured to generate representative image data describing a location one or more of the physiological landmarks of subject heart 22 within a multi-dimensional space (e.g., a space having three or more dimensions).

Processing circuitry 132 may be configured to provide the representative image data generated by the first algorithm to a second algorithm. The second algorithm may be configured to use the representative image data to locate one or more target sites on subject heart 22. A target site may be location defined on subject heart 22 where conduction system pacing is likely to occur when electrode 124 establishes electrical communication with tissue of subject heart 22 within the target site. The second algorithm may include a machine learning algorithm (e.g., an artificial intelligence (AI) algorithm) trained with a data set indicative or one or more previous locations defined on an anatomical heart where successful or unsuccessful conduction system pacing occurred as a result of establishing electrical communication with the anatomical heart at the one or more locations defined by the anatomical heart. The machine learning algorithm may be configured to receive an input vector indicative of the physiological landmarks of subject heart 22 from the first algorithm, and define one or more target sites on subject heart 22 using the input vector and the previous locations defined within the training data set.

In examples, processing circuitry 132 is configured to generate a representative image of subject heart 22 on visual display 142 using the representative image data generated by the first algorithm. Processing circuitry 132 may be configured to provide a representative image which includes information identified by the first algorithm, such as the physiological landmarks of subject heart 22 identified, or other information. In some examples, processing circuitry 132 may be configured to evaluate a confidence level of the visual fidelity and/or positional accuracy of a representative image generated or to be generated using the representative image data available from the first algorithm. Processing circuitry 132 may be configured to provide an output to a clinician suggesting additional steps which may improve the visual fidelity and/or positional accuracy. For example, processing circuitry 132 may be configured to evaluate a confidence level describing the placement of an individual physiological landmark of subject heart 22 within the representative image relative to other physiological landmarks, and recommend one or more additional steps such as use of a contrast dye (e.g., a dye shot"), the capture of additional images of subject heart 22, one or more different viewing angles for imaging device 130 (e.g., recommending an LAO or RAO angulation), using a different imaging mode (e.g., CINE fluorography), placing a fluoro-visible marker on the patient, changing a pacing output of electrode 124 (e.g., from bipolar to unipolar, or vice-versa), or some other steps intended to improve the confidence level.

As discussed, processing circuitry may be configured to determine electrode position data indicative of a position of electrode 124 within subject heart 22 using at least in part the image data generated imaging device 130. In examples, processing circuitry 132 is configured to determine the electrode position data using the image data received and an image recognition algorithm (e.g., the first algorithm) to discern apparatus portion 120 and/or electrode 124 from among the physiological landmarks identified for subject heart 22. Processing circuitry 132 may be configured to determine a position of electrode 124 relative to the physical landmarks identified, or some other anatomical structure of subject heart 22. In some examples, processing circuitry 132 is configured to determine the electrode position data based on a signal received from electrode 124. For example, as discussed, when electrode 124 is in electrical communication with tissues of subject heart 22, processing circuitry 132 may be configured to assess a position where electrode 124 is likely to be located within subject heart 22 based on a comparison of the received signal from electrode 124 with signals expected from various locations within the conduction system of subject heart 22. Processing circuitry 132 may be configured to generate a representative image of electrode 124 on visual display 142 based on the assessed position of electrode 124. In examples, processing circuitry 132 is configured to indicate the assessed position of electrode 124 within (e.g., as a pictorial element of, or superimposed on) the representative image of subject heart 22 displayed on visual display 142.

In examples, processing circuitry 132 is configured for operable connection with a medical device 150 (e.g., via communication link 156) configured to establish electrical communication with a torso of patient 16. Medical device 150 may include a device lead 152 and device circuitry 154. Medical device 150 may be configured to sense electrical and/or mechanical activity of subject heart 22 within patient 16. In examples, medical device 150 includes a plurality of electrodes 159 configured to establish electrical communication with the torso of patient 16 and generate one or more signals indicative of the electrical and/or mechanical activity of subject heart 22. Medical device 150 may be, for example, a wearable belt or vest (e.g., an ECG belt or vest) including plurality of electrode 159. Processing circuitry 132 may be configured to receive the one or more electrical signals from medical device 150. In examples, processing circuitry 132 is configured to base the likelihood of success for given electrode position data indicative of the position of electrode 124 in subject heart 22 based at least in part on the one or more indicative signals received from medical device 150. In some examples, processing circuitry 132 is configured to utilize at least in part the one or more indicative signals from medical device 150 to generate the representative image of subject heart 22, apparatus portion 120, and/or electrode 124 on visual display 142. For example, processing circuitry 132 and/or medical device 150 may be configured to determine one or more pseudo-electric vectors (PEVs) representing electrical forces generated by subject heart 22. Processing circuitry 132 may be configured to generate the representative image of subject heart 22, apparatus portion 120, and/or electrode 124 using the one or more PEVs. In some examples, imaging device 130 is configured to receive the one or more indicative signals from medical device 150 to generate the image of subject heart 22, apparatus portion 120, and/or electrode 124 on display apparatus 138.

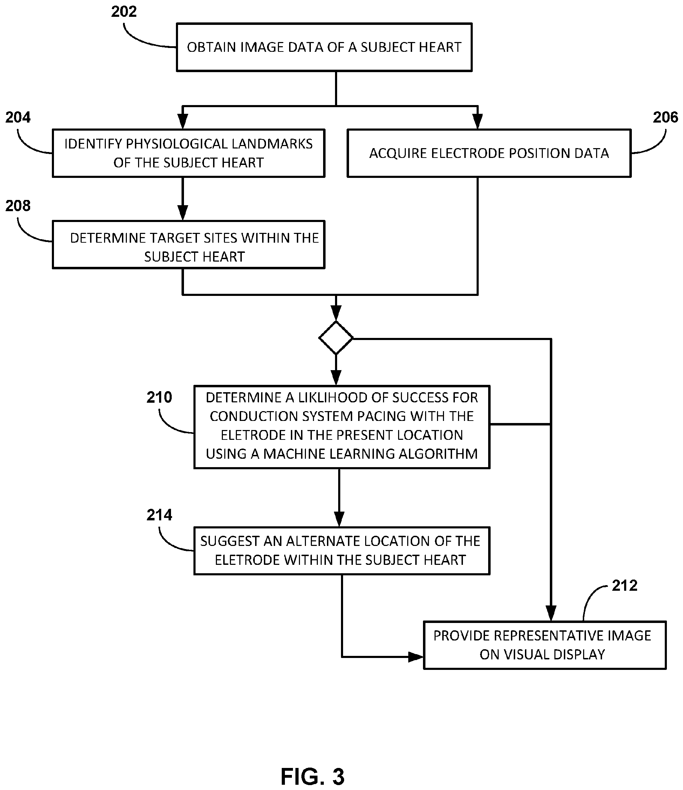

FIG. 3 is a flowchart illustrating an example technique of determining a likelihood of success for conduction system pacing when an electrode 124 is in electrical communication with a subject heart 22. The example technique may be executed by components of medical system 100 and/or the components of other systems.

Processing circuitry 132 may obtain image data associated with subject heart 22 of patient 16 (202). The image data may be further associated with an electrode 124 supported by an apparatus 112 within subject heart 22. The image data may include one or more frames representing images of subject heart 22 and/or subject heart 22 and electrode 124. In examples, the image data in generated by imaging device 130 and communicated to processing circuitry 132 via communication link 136. In examples, the image data is generated by image device 146. In some examples, imaging device 130 displays an image of subject heart 22 and/or subject heart 22 and electrode 124 on display apparatus 138, and image device 146 generates the image data when display apparatus 138 is within a field-of-view of image device 146.

Processing circuitry 132 may identify physiological landmarks of subject heart 22 using the image data (204). Processing circuitry 132 may identify the physiological landmarks using a first algorithm (e.g., an image recognition algorithm) trained to identify anatomical structures of an anatomical heart, such as atria, ventricles, vena cava, anatomical valves, and other various regions of anatomical hearts. The first algorithm may conduct image segmentation of the image of subject heart 22 using the image data provided by processing circuitry 132 (e.g., from image device 146 and/or imaging device 130). In examples, processing circuitry 132 receives imaging data representative of subject heart 22 and/or subject heart 22 and electrode 124 and generates representative image data using the physiological landmarks identified by the first algorithm. Processing circuitry 132 may receive imaging data representative of one or more two-dimensional images of subject heart 22, and using the first algorithm to identify physiological landmarks of subject heart 22, generate representative image data describing a three-dimensional representation of subject heart 22.

Processing circuitry 132 may acquire electrode location data indicative of a position of electrode 124 within subject heart 22 (206). Processing circuitry may acquire the electrode location data using at least in part the image data generated imaging device 130 and/or image device 146. In examples, processing circuitry 132 is configured to acquire the electrode position data by generating at least some portion of the electrode position data using the image data received. In examples, processing circuitry 132 is configured to acquire the electrode position data by receiving one or more signals (e.g., from electrode 124, from apparatus 112, and/or from another system configured to determine a position of the electrode within the heart of a patient) using the one or more signals. Processing circuitry 132 may use an image recognition algorithm (e.g., the first algorithm) to discern apparatus portion 120 and/or electrode 124 from among the physiological landmarks identified for subject heart 22. Processing circuitry 132 may assess a position of electrode 124 relative to the physical landmarks identified, or some other anatomical structure of subject heart 22.

In some examples, electrode 124 establishes electrical communication with subject heart 22 and provides an signal to processing circuitry 132. Processing circuitry 132 may determine the electrode position data indicative of the position of electrode 124 based on a signal received from electrode 124 or based on a cardiac response (e.g., an electrical activation time) of subject heart 22 when electrode 124 establishes electrical communication with subject heart 22. Processing circuitry 132 may receive one or more signals indicating the cardiac response from medical device 150, or another device configured to monitor subject heart 22. Processing circuitry 132 may assess a location where electrode 124 is likely to be located within subject heart 22 based on a comparison of the received signal from electrode 124 and/or the cardiac response with historical signals received when an electrode established electrical communication at various locations within the conduction system of anatomical hearts (e.g., during past implantation and/or conduction system pacing procedures).

In examples, processing circuitry 132 may determine the location of one or more target sites on subject heart (208). In examples, processing circuitry 132 provides the representative image data generated by the first algorithm to a second algorithm. The second algorithm may use the representative image data provided by the first algorithm to locate the one or more target sites. The second algorithm may use a machine learning algorithm trained with a data set indicative or one or more previous locations defined on an anatomical heart where successful or unsuccessful conduction system pacing occurred as a result of establishing electrical communication with the anatomical heart at the one or more locations defined by the anatomical heart. The machine learning algorithm may receive an input vector indicative of the physiological landmarks of subject heart 22 identified by the first algorithm and provide an output vector defining the one or more target sites on subject heart 22. The one or more target sites may be indicative of, for example, an LV endocardial pacing location, an epicardial pacing location, a location on the coronary sinus branch, or some other location of subject heart 22.

Processing circuitry 132 may determine a likelihood of success for conduction system pacing of subject heart 22 based on the electrode position data of electrode 124 and target sites identified for subject heart 22 (210). In examples, processing circuitry 132 determines the likelihood of success based the electrode position data and the one or more target sites. In some examples, processing circuitry 132 determine the likelihood of success at least in part using the one or more signals received from medical device 150, electrode 124, and/or another device configured to indicate a cardiac response of subject heart 22.

Processing circuitry 132 may determine the likelihood of success using a machine learning algorithm trained with a data set based on one or more past procedures wherein an electrode was positioned within an anatomical heart for the generation of conduction system pacing. In examples, the machine learning algorithm is a portion of the second algorithm. The training set may comprise, for examples, a plurality of individual input vectors and a plurality of individual output vectors, which each output vector corresponding to an individual input vector. The individual input vectors may include elements descriptive of the location of an electrode on an anatomical heart (e.g., a human heart), the relative location of the electrode compared to one or more physiological markers of the anatomical heart, a signal characteristics of a signal generated by the electrode when in electrical communication with the anatomical heart, one or more signals from a medical device providing the cardiac response of the anatomical heart, or other descriptive elements. The output vector may include one or more elements indicative of the likelihood of success of conduction system pacing for the anatomical heart. The machine learning algorithm may be trained to receive an individual input vector from in the training set and substantially map the individual input vector onto a space defined by the individual output vectors. Examples of machine learning algorithms that may be so configured to perform aspects of this disclosure include classifiers and non-classification ML models, artificial neural networks ("NNs"), linear regression models, logistic regression models, decision trees, support vector machines ("SVM"), Naive or a non-Naive Bayes network, k-nearest neighbors ("KNN") models, deep learning (DL) models, k-means models, clustering models, random forest models, or any combination thereof. Depending on the implementation, the ML models may be supervised, unsupervised or in some instances, a hybrid combination (e.g., semi-supervised).

Processing circuitry 132 may generate an in-situ input vector and provide the in-situ input vector to the trained machine learning algorithm in order to determine the likely of success for conduction system pacing of subject heart 22 when electrode 124 is in the position indicated by the electrode position data. Processing circuitry may generate the in-situ input vector using imaging data from imaging device 130 and/or image device 146, signals from electrode 124, one or more signals from medical device 150, and/or other portions of medical system 100. The trained machine learning algorithm may receive the in-situ input vector and subsequently indicate a likelihood of success for conduction system pacing based on the in-situ input vector provided by processing circuitry 132.

Processing circuitry 132 may indicate the likelihood of success determined using the machine learning algorithm in a format viewable by a clinician (212). For example, processing circuitry 132 may indicate the likelihood of success using visual display 142. Processing circuitry 132 may repeat one or more of obtaining image date, identifying physiological landmarks of subject heart 22, acquiring electrode position data for electrode 124, determining target sites on subject heart 22, and determining the likelihood of success, such that when a location of electrode 124 within subject heart 22 is altered (e.g., by a clinician), processing circuitry 132 updates visual display 142 to indicate a revised likelihood of success based on the altered location of electrode 124. Processing circuitry 132 may repeat the steps discussed above either periodically based on an established refresh rate, in response to a command input by a clinician, or based on some other criteria.

Processing circuitry 132 may generate a representative image of subject heart 22 on visual display 142 using the physiological landmarks of subject heart 22 identified using the image data generated by imaging device 130 and/or image device 146. Processing circuitry 132 may include (e.g., superimpose) the determined target sites on the representative image generated on visual display 142. In examples, processing circuitry 132 further generates a representative image of apparatus portion 120 and/or electrode 124 on visual display 142, based on an electrode position data indicative of a position of electrode 124 within subject heart 22. Processing circuitry 132 may include the representative image of apparatus portion 120 and/or electrode 124 within the representative image of heart 22, such that a clinician viewing visual display 142 may assess the location of electrode 124 relative to the target sites provided on the representative image of heart 22.

In some examples, processing circuitry 132 suggests and/or indicates an alternate location for electrode 124 within subject heart 22 (214). For example, processing circuitry 132 may suggest the alternate location when the likelihood of success determined for the electrode position data for electrode 124 is below a threshold. Processing circuitry 132 may suggest the alternate location based on the electrode location data acquired for electrode 124 and the one or more target sites determined for subject heart 22. For example, processing circuitry 132 may suggest an alternate target site based on the proximity of electrode 124 and the alternate target site, based on signals received from electrode 124, one or more signals from medical device 150, or some other criteria.

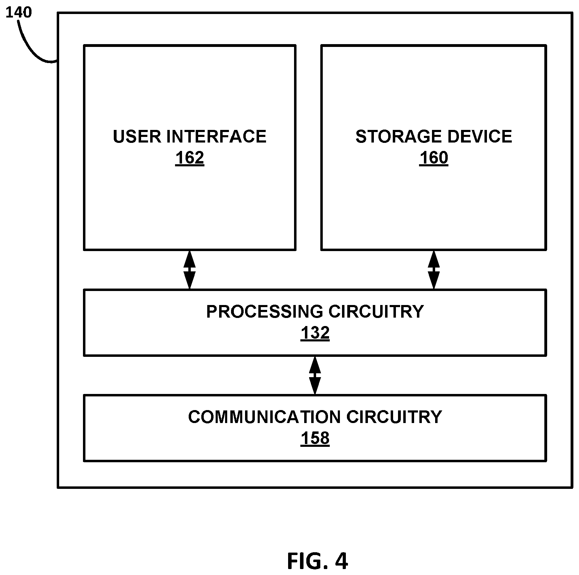

In examples, processing circuitry 132 is configured to assess the delivery of conduction system pacing to subject heart 22 by electrode 124 and provide feedback to the machine learning algorithm. For example, processing circuitry 132 may be configured to evaluate the delivery of conduction system pacing using at least one of the medical device 150, a signal from electrode 124, or an input from a clinician provided via a user interface (e.g., user interface 162 (FIG. 4)). Processing circuitry 132 may be configured to provide a quality indicator reflective of the conduction system pacing delivered with electrode 124 in the position within subject heart 22 indicated by the electrode position data. The machine learning algorithm may be configured to engage in machine learning (e.g., supervised, unsupervised, and/or reinforcement learning) using the quality indicator. The quality indicator may be, for example, a vector of features constituting a description of the conduction system pacing delivered by electrode 124. In examples, processing circuitry 132 is configured to generate an training input vector including elements descriptive of the electrode position data indicative of the position of electrode 124 within subject heart 22, a relative location of electrode 124 compared to one or more physiological markers of subject heart 22, signal characteristics of a signal generated by electrode 124 when in electrical communication with subject heart 22, one or more signals from medical device 150, or other descriptive elements. Processing circuitry 132 may be configured to define a training output vector descriptive of the quality indicator. In examples, processing circuitry is configured to associate the training input vector and the training output vector and update the training set used to train the machine algorithm. Processing circuitry 132 may be configured to cause the machine learning algorithm to engage in the machine learning using the updated training set.

FIG. 4 is a block diagram illustrating an example configuration of components of external device 140. As discussed, external device 140 may include at least some portion of processing circuitry 132. External device 140 may be a portable device. In examples, external device 140 may include image device 146 and/or visual display 142 (FIG. 2). External device 140 may be configured such that a clinician may reposition external device 140 to capture an image of the video screen (e.g., using image device 146) and generate image data representative of the image, and subsequently provide the image data to processing circuitry 132. Processing circuitry 132 may use the image data to provide an output (e.g., via visual display 142) indicating a likelihood of success for conduction system pacing as disclosed herein.

In the example of FIG. 4, external device 140 includes processing circuitry 132, communication circuitry 158, storage device 160, and a user interface 162. Processing circuitry 132 may include one or more processors that are configured to implement functionality and/or process instructions for execution within external device 140. For example, processing circuitry 132 may be capable of processing instructions stored in storage device 160. Processing circuitry 132 may include, for example, microprocessors, DSPs, ASICs, FPGAs, or equivalent discrete or integrated logic circuitry, or a combination of any of the foregoing devices or circuitry. Accordingly, processing circuitry 132 may include any suitable structure, whether in hardware, software, firmware, or any combination thereof, to perform the functions ascribed herein to processing circuitry 132.

Communication circuitry 158 may include any suitable hardware, firmware, software or any combination thereof for communicating with another device, such as imaging device 130, apparatus 112, medical device 150, and/or others. Communication circuitry 158 may be configured to receive and/or send communications under the control of processing circuitry 132. Communication circuitry 158 may be configured to send and receive communications via communication links 136, 144, 146. In examples, communication circuitry 158 is configured to send communications to and/or receive communications, including downlink and uplink telemetry, from components not shown in FIG. 3. Communication circuitry 158 may be configured to transmit or receive signals via inductive coupling, electromagnetic coupling, Near Field Communication (NFC), Radio Frequency (RF) communication, Bluetooth, Wi-Fi, or other proprietary or non-proprietary wireless communication schemes. Communication circuitry 158 may also be configured to communicate with devices such as imaging device 130, apparatus 112, medical device 150, and/or others via any of a variety of forms of wired and/or wireless communication and/or network protocols.

Storage device 160 may be configured to store information within external device 140 during operation. Storage device 160 may include a computer-readable storage medium or computer-readable storage device. In some examples, storage device 160 includes one or more of a short-term memory or a long-term memory. Storage device 160 may include, for example, RAM, DRAM, SRAM, magnetic discs, optical discs, flash memories, or forms of EPROM or EEPROM. Storage device 160 may be used to store at least a portion of the image data generated by imaging device 130 and/or image device 146, at least a portion of the one or more signals generated by medical device 150, and/or at least a portion of a signal received from electrode 124. In examples, storage device 160 is used to store data indicative of instructions for execution by processing circuitry 132. Storage device 160 may be used by software or applications running on external device 140 to temporarily store information during program execution.

A user, such as a clinician, may interact with external device 140 through user interface 162. User interface 162 may include visual display 142 (FIG. 2), with which processing circuitry 132 may present information related the likelihood of success for conduction system pacing based on electrode position data for electrode 124, cardiac signals received from medical device 150 and/or electrode 124, representative images of subject heart 22, apparatus portion 120, and/or electrode 124, the location of one or more target sites on subject heart 22, recommendations for the repositioning of electrode 124, and other information. In addition, user interface 162 may include an input mechanism to receive input from clinician. The input mechanisms may include, for example, any one or more of buttons, a keypad (e.g., an alphanumeric keypad), a peripheral pointing device, a touch screen, or another input mechanism that allows the user to navigate through user interfaces presented by processing circuitry 132 of external device 140 and provide input. In other examples, user interface 162 also includes audio circuitry for providing audible notifications, instructions or other sounds to the user, receiving voice commands from the user, or both.

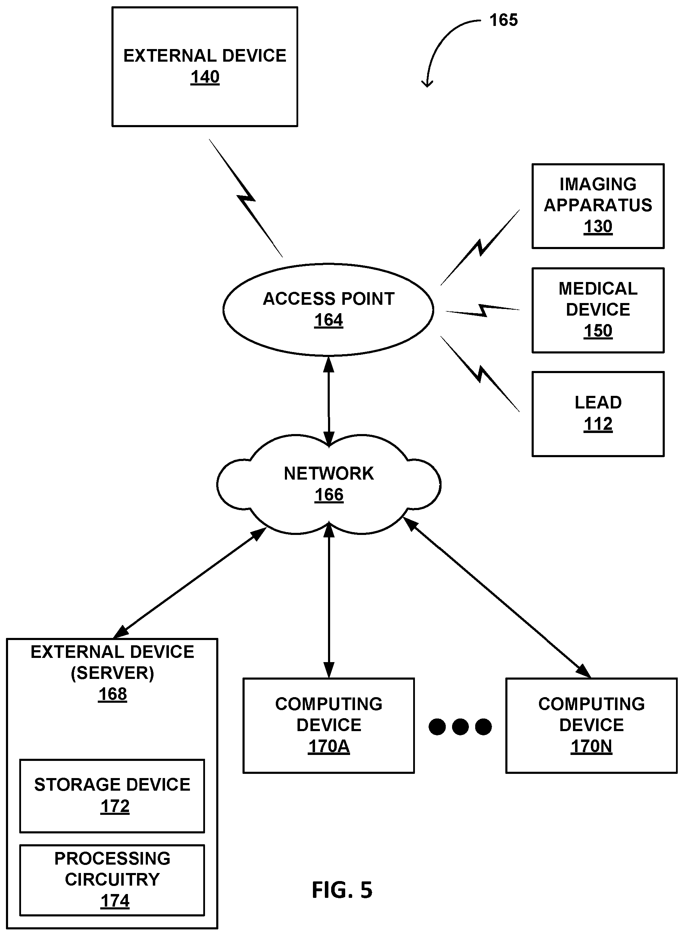

FIG. 5 is a block diagram illustrating an example system 165 including external device 140, an access point 164, a network 166, a server 168, and one or more other computing devices 170A-170N. System 165 may include imaging device 130, medical device 150, and/or apparatus 112. External device 140, imaging device 130, medical device 150, and/or apparatus 112 may be coupled to network 166 (e.g., via access point 164) in accordance with one or more techniques described herein. For example, external device 140 may use communication circuitry 158 communicate with access point 164 via a hard-line or wireless connection. In the example of FIG. 5, external device 140, imaging device 130, medical device 150, apparatus 112, access point 164, server 168, and/or computing devices 170A-170N are interconnected and may communicate with each other through network 166. Access point 164 may include a device that connects to network 166 via any of a variety of connections, such as telephone dial-up, digital subscriber line (DSL), or cable modem connections. In other examples, access point 164 may be coupled to network 166 through different forms of connections, including wired or wireless connections.

Server 168 may be configured to provide a secure storage site for data that has been collected from external device 140, imaging device 130, medical device 150, and/or apparatus 112. In some cases, server 168 may assemble data in web pages or other documents for viewing by trained professionals, such as clinicians, via computing devices 170A-170N. In examples, server 168 may comprise one or more servers, a cloud, one or more databases, and/or a data center. Server 168 may include storage device 172 (e.g., a memory device) to, for example, store data retrieved from external device 140, imaging device 130, medical device 150, and/or apparatus 112. External device 140 may include processing circuitry 174 including one or more processors that are configured to implement functionality and/or process instructions for execution within server 168. For example, processing circuitry 174 may be capable of processing instructions stored in storage device 172. Processing circuitry 174 may include, for example, microprocessors, DSPs, ASICs, FPGAs, or equivalent discrete or integrated logic circuitry, or a combination of any of the foregoing devices or circuitry. Storage device 172 may include a computer-readable storage medium or computer-readable storage device. In some examples, storage device 172 includes one or more of a short-term memory or a long-term memory, such as RAM, DRAM, SRAM, magnetic discs, optical discs, flash memories, or forms of EPROM or EEPROM.

In examples, processing circuitry 174 includes or is a portion of processing circuitry 132. Processing circuitry 174 may be configured to perform all of some portion of the functionality described with respect to processing circuitry 132. External device 140, imaging device 130, medical device 150, and/or apparatus 112 may be configured to provide data to server 168 to enable processing circuitry 174 to perform any portion of the functionality described with respect to processing circuitry 132. For example, processing circuitry 174 may be configured conduct image recognition for the identification of physiological landmarks of subject heart 22 (FIG. 2) based on image data provided to server 166 from imaging device 130 and/or image device 146. Processing circuitry 174 may be configured to generate representative image data of subject heart 22 and provide the representative image data to visual display 142 (FIG. 2). Processing circuitry 174 may be configured to determine a likelihood of success for conduction system pacing of subject heart 22 using a machine learning algorithm (e.g., an artificial intelligence (AI) algorithm) trained with a training data set indicative of successful electrode placements in conduction system pacing (CSP) procedures performed in the past on previous CSP patients, and may be configured to provide an output indicating the likelihood of success to a clinician using visual display 142.

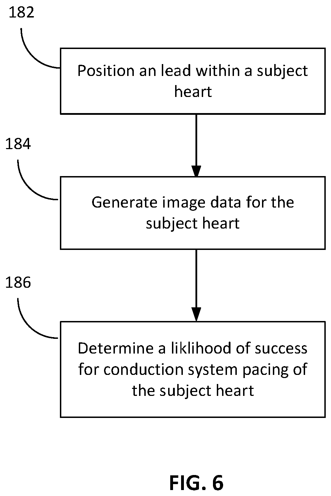

A technique for positioning an electrode 124 within a subject heart 22 is illustrated in FIG. 6. Although the technique is described mainly with reference to medical system 10, 100, 165 of FIGS. 1-5, the technique may be applied to other medical systems in other examples.

The technique includes positioning an apparatus 12, 112 within a subject heart 22 of a patient 16 (182). Apparatus 12, 112 includes electrode 124 and may include lead body 118, apparatus portion 120, and/or apparatus circuitry 134. Electrode 124 may be configured to establish electrical communication with tissues of subject heart 22. In examples, apparatus 12, 112 includes an apparatus portion 120 supporting electrode 124. At least apparatus portion 120 may be steerable within subject heart 22.

The technique includes generating image data representative of an image of subject heart 22 (184). In examples, the technique includes generating an image of subject heart 22 using an imaging device 130 and producing the image data using the image of subject heart 22 generated. In examples, the technique includes generating the image data using external device 140. External device 140 may include image device 146 defining a field-of-view. The technique may include generating the image data by positioning external device 140 such that image is within the field-of view defined.

The technique includes providing the image data to processing circuitry 132, 174 and determining a likelihood of successful conduction system pacing for subject heart 22 by electrode 124 using a machine learning algorithm (186). The technique may include acquiring electrode position data indicative of a position of electrode 124 within subject heart 22 using the image data. In examples, processing circuitry 132 is operably coupled to electrode 124, and processing circuitry 132 determines the electrode position data at least in part using a signals from electrode 124. In examples, processing circuitry 132 is operably coupled to a medical device 150 configured to establish electrical communication with a torso of patient 16, and processing circuitry 132 determines the electrode position data at least in part using one or more signals from medical device 150.

In some examples, the technique includes recognizing, using processing circuitry 132, 174, physiological landmarks of subject heart 22 using the image data. Processing circuitry 132, 174 may recognize the physiological landmarks using an image recognition algorithm trained to identify physiological landmarks and/or anatomical structures of subject heart 22 represented within image data, such as atria, ventricles, vena cava, anatomical valves, various regions of a human heart, and other physiological landmarks. Processing circuitry 132 may be configured to determine one or more target sites on subject heart 22 using the identified physiological landmarks. In examples, processing circuitry 132 determines a position of electrode 124 relative to the target sites. In some examples, processing circuitry 132 generates a representative image of subject heart 22, electrode 124, and/or the target sites on a visual display 142. The technique may include refresh the representative image, such that when a position of electrode 124 within subject heart 22 is altered (e.g., by a clinician), processing circuitry 132 updates visual display 142 to indicate a new position of electrode 124 relative to the target sites and/or the physiological landmarks of subject heart 22.

The technique may using processing circuitry 132 to determine the likelihood of success for conduction system pacing of subject heart 22 based on the electrode position data for electrode 124 and the target sites and/or physiological landmarks of subject heart 22. The likelihood of success may be based on one or more past procedures wherein an electrode was positioned within an anatomical heart for the generation of conduction system pacing. Processing circuitry 132 may determine the likelihood of success using a machine learning algorithm (e.g., an artificial intelligence (AI) algorithm) trained with a data set reflecting the past procedures. The trained machine learning algorithm may be configured to subsequently receive data indicative of the present position of electrode 124 relative to the target sites and/or physiological landmarks identified for subject heart 22 and indicate a likelihood of success for conduction system pacing based on the training conducted using the training data set.

In examples, the technique includes processing circuitry 132 utilizing a first algorithm to identify physiological landmarks of subject heart 22 and utilizing a second algorithm to determine the likelihood of success for conduction system pacing based at least in part on the physiological landmarks identified by the first algorithm. The first algorithm may be an image recognition algorithm trained to identify anatomical structures of an anatomical heart such as subject heart 22. In examples, the first algorithm is configured to generate representative image data describing a location one or more of the physiological landmarks of subject heart 22 within a multi-dimensional space (e.g., a space having three or more dimensions). The technique may include processing circuitry providing the representative image data generated by the first algorithm to a second algorithm. The second algorithm may use the representative image data to locate one or more target sites on subject heart 22. In examples, second algorithm receives an input vector indicative of the physiological landmarks of subject heart 22 from the first algorithm, and defines one or more target sites on subject heart 22 using the input vector.

The technique may include assessing the delivery of conduction system pacing to subject heart 22 by electrode 124 and providing feedback to the machine learning algorithm. In examples, processing circuitry 132 evaluates the delivery of conduction system pacing using at least one of the medical device 150, a signal from electrode 124, or an input from a clinician provided via user interface 162. Processing circuitry 132 may be configured to provide a quality indicator reflective of the conduction system pacing delivered with electrode 124 in the position within subject heart 22 indicated by the electrode position data. The technique may include generating, using processing circuitry 132, a training input vector including elements descriptive of the electrode position data for electrode 124 within subject heart 22, a relative location of electrode 124 compared to one or more physiological markers of subject heart 22, signal characteristics of a signal generated by electrode 124 when in electrical communication with subject heart 22, one or more signals from medical device 150, or other descriptive elements. The technique may include defining, using processing circuitry 132, a training output vector descriptive of a quality indicator. In examples, processing circuitry 132 associates the training input vector and the training output vector and updates the training set used to train the machine algorithm. Processing circuitry 132 may cause the machine learning algorithm to engage in the machine learning using the updated training set.

## Claims

1. A medical system (100) comprising:
an imaging device (130) configured to generate an image representative of a subject heart (22) of a patient (16) and representative of an apparatus portion (120) within the subject heart, wherein the apparatus portion is at least a portion of an apparatus (12), and wherein the apparatus portion includes an electrode (124); and
processing circuitry (132) configured to:
receive image data representative of the image of a subject heart and the apparatus portion within the subject heart,
acquire electrode position data indicative of a position of the electrode within the subject heart, and
apply a machine learning algorithm to generate an output readable by a clinician indicating a likelihood of successful conduction system pacing based on the electrode position data,
wherein the machine learning algorithm is trained using a training data set indicative of one or more positions of an implanted electrode within an anatomical heart when the implanted electrode successfully and unsuccessfully established conduction system pacing of the anatomical heart.

2. The medical system of claim 1, wherein the processing circuitry is configured to:
map one or more target sites (14, 15) of the subject heart using the received image data, wherein the one or more target sites describe a location on the subject heart;
assess the position of the electrode within the subject heart using the electrode position data;
compare the assessed position of the electrode to the one or more target sites; and
generate the output based on the comparison of the assessed position of the electrode and the one or more target sites.

3. The medical system of claim 1 or 2, wherein the processing circuitry is configured to:
receive a signal indicative of an electrical signal sensed via the electrode, and
base the output on the signal indicative of the electrical signal.

4. The medical system of any one of claims 1 to 3, further comprising a medical device (150) operably coupled to the processing circuitry, wherein:
the medical device is configured to establish electrical communication with a torso of the patient (16),
the medical device is configured to generate a signal representative of a physiological parameter of the subject heart, and
the processing circuitry is configured to base the output on the signal representative of the physiological parameter of the subject heart,
particularly wherein the medical device comprises one or more device electrodes configured to establish electrical communication with the torso of the patient.

5. The medical system of any one of claims 1 to 4, wherein the processing circuitry is configured to extract features of the subject heart using the image data and an image recognition algorithm, wherein the image recognition algorithm is trained using training image data representing a physiological heart separate from the subject heart.

6. The medical system of claim 5, wherein the processing circuitry is configured to acquire the electrode position data using the extracted features; and/or
wherein the processing circuitry is configured to:
determine a confidence level based on the extracted features, wherein the confidence level is based on at least one of a visual fidelity of the extracted features or a positional accuracy of the extracted features; and
provide a procedural instruction to the output readable by the clinician to improve the confidence level.

7. The medical system of any one of claims 1 to 6, further comprising a visual display (142),
wherein the processing circuitry is configured to extract features of the subject heart using the received image data and produce a representative image of the subject heart on the visual display using the extracted features, particularly wherein the processing circuitry is configured to indicate a representative electrode location on the representative image, wherein the representative electrode location is representative of the position of the electrode indicated by the electrode location data.

8. The medical system of any one of claims 1 to 7, further comprising an external device (140),
wherein a portion of the processing circuitry is contained within the external device, and wherein the external device includes an image device configured to produce the image data representative of the image of the subject heart and the apparatus portion generated by the imaging device, particularly wherein the image device defines a field-of-view, and wherein the image device is configured to produce the image data when the image of the subject heart and the apparatus portion are within the field-of view.

9. The medical system of any one of claims 1 to 8, wherein the processing circuitry is configured to
receive a signal indicative of an electrical signal of the electrode, and
update the training set of the machine learning algorithm using the indicative signal and the electrode position data.

10. The medical system of any one of claims 1 to 9, wherein the processing circuitry is configured to:
determine a dimension of the apparatus portion based on one or more physical structures on the apparatus portion recognizable by the processing circuitry when the apparatus portion is positioned within the subject heart; and
generate some portion of the electrode position data based on the dimension.

11. The medical system of any one of claims 1 to 10, further comprising the apparatus portion, wherein the apparatus portion is configured to generate an electrical signal when the electrode of the apparatus portion is in electrical communication with tissues of the subject heart, wherein the apparatus portion is configured to be steerable when the apparatus portion is within the subject heart.

12. The medical system of any one of claims 1 to 6, further comprising:
a medical device configured to establish electrical communication with a torso of a patient, wherein the medical device is configured to generate a signal representative of a physiological parameter of a subject heart of the patient; and
an apparatus including an apparatus portion supporting an electrode, wherein the apparatus is configured to generate an electrical signal when the electrode is in electrical communication with tissues of the subject heart, wherein the apparatus portion is configured to be steerable when the apparatus portion is within the subject heart;
a visual display;
wherein the processing circuitry is further configured to:
extract features of the subject heart using the received image data and produce a representative image of the subject heart on the visual display using the extracted features;
receive the representative signal from the medical device;

13. The medical system of claim 12, wherein the processing circuitry is configured to:
map one or more target sites of the subject heart using the received image data, wherein the one or more target sites describe a location on the subject heart;
assess the position of the electrode within the subject heart using the electrode position data;
compare the assessed position of the electrode to the one or more target sites; and
generate the output based on the comparison of the assessed position of the electrode and the one or more target sites; and/or.
wherein the processing circuitry is configured to:
receive a signal indicative of the electrical signal of the electrode, and
base the output on the signal indicative of the electrical signal.

## Patentansprüche

1. Medizinisches System (100), umfassend:
eine Bildgebungsvorrichtung (130), die konfiguriert ist, um ein Bild zu generieren, das ein betreffendes Herz (22) eines Patienten (16) darstellt und einen Geräteabschnitt (120) innerhalb des betreffenden Herzens darstellt, wobei der Geräteabschnitt mindestens ein Abschnitt eines Geräts (12) ist und wobei der Geräteabschnitt eine Elektrode (124) einschließt; und
Verarbeitungsschaltung (132), die konfiguriert ist zum:
Empfangen von Bilddaten, die ein Bild eines betreffenden Herzens und des Geräteabschnitts innerhalb des betreffenden Herzens darstellen,
Erfassen von Elektrodenpositionsdaten, die eine Position der Elektrode innerhalb des betreffenden Herzens angeben, und
Anwenden eines Machine-Learning-Algorithmus zum Generieren einer für einen Kliniker lesbaren Ausgabe, die eine Wahrscheinlichkeit einer erfolgreichen Reizleitungssystemstimulation basierend auf den Elektrodenpositionsdaten angibt,
wobei der Machine-Learning-Algorithmus unter Verwendung eines Trainingsdatensatzes trainiert wird, der eine oder mehrere Positionen einer implantierten Elektrode innerhalb eines anatomischen Herzens angibt, wenn die implantierte Elektrode erfolgreich und erfolglos eine Reizleitungssystemstimulation des anatomischen Herzens eingerichtet hat.

2. Medizinisches System nach Anspruch 1, wobei die Verarbeitungsschaltung konfiguriert ist zum:
Abbilden einer oder mehrerer Zielstellen (14, 15) des betreffenden Herzens unter Verwendung der empfangenen Bilddaten, wobei die eine oder die mehrere Zielstellen eine Position an dem betreffenden Herzen beschreiben;
Ermitteln der Position der Elektrode innerhalb des betreffenden Herzens unter Verwendung der Elektrodenpositionsdaten;
Vergleichen der ermittelten Position der Elektrode mit der einen oder den mehreren Zielstellen und
Generieren der Ausgabe basierend auf dem Vergleich der ermittelten Position der Elektrode mit der einen oder den mehreren Zielstellen.

3. Medizinisches System nach Anspruch 1 oder 2, wobei die Verarbeitungsschaltung konfiguriert ist zum:
Empfangen eines Signals, das ein über die Elektrode erfasstes elektrisches Signal angibt, und
Basieren der Ausgabe auf dem Signal, das das elektrische Signal angibt.

4. Medizinisches System nach einem der Ansprüche 1 bis 3, ferner umfassend eine medizinische Vorrichtung (150), die mit der Verarbeitungsschaltung wirkgekoppelt ist, wobei:
die medizinische Vorrichtung konfiguriert ist, um eine elektrische Verbindung mit dem Oberkörper des Patienten (16) herzustellen,
die medizinische Vorrichtung konfiguriert ist, um ein Signal zu generieren, das einen physiologischen Parameter des betreffenden Herzens darstellt, und
die Verarbeitungsschaltung konfiguriert ist, um die Ausgabe auf dem Signal zu basieren, das den physiologischen Parameter des betreffenden Herzens darstellt,
insbesondere wobei die medizinische Vorrichtung eine oder mehrere Vorrichtungselektroden umfasst, die konfiguriert sind, um eine elektrische Verbindung mit dem Oberkörper des Patienten herzustellen.

5. Medizinisches System nach einem der Ansprüche 1 bis 4, wobei die Verarbeitungsschaltung konfiguriert ist, um Merkmale des betreffenden Herzens unter Verwendung der Bilddaten und eines Bilderkennungsalgorithmus zu extrahieren, wobei der Bilderkennungsalgorithmus unter Verwendung von Trainingsbilddaten trainiert wird, die ein physiologisches Herz darstellen, das von dem betreffenden Herzen getrennt ist.

6. Medizinisches System nach Anspruch 5, wobei die Verarbeitungsschaltung konfiguriert ist, um die Elektrodenpositionsdaten unter Verwendung der extrahierten Merkmale zu erfassen; und/oder
wobei die Verarbeitungsschaltung konfiguriert ist zum:
Bestimmen eines Konfidenzniveaus basierend auf den extrahierten Merkmalen, wobei das Konfidenzniveau auf mindestens einem von einer visuellen Wiedergabetreue der extrahierten Merkmale oder einer Positionsgenauigkeit der extrahierten Merkmale basiert; und
Bereitstellen einer für den Kliniker lesbaren Verfahrensanweisung an die Ausgabe, um das Konfidenzniveau zu verbessern.

7. Medizinisches System nach einem der Ansprüche 1 bis 6, ferner umfassend eine visuelle Anzeige (142),
wobei die Verarbeitungsschaltung konfiguriert ist, um Merkmale des betreffenden Herzens unter Verwendung der empfangenen Bilddaten zu extrahieren und ein repräsentatives Bild des betreffenden Herzens auf der visuellen Anzeige unter Verwendung der extrahierten Merkmale zu erzeugen, insbesondere wobei die Verarbeitungsschaltung konfiguriert ist, um eine repräsentative Elektrodenposition auf dem repräsentativen Bild anzugeben, wobei die repräsentative Elektrodenposition die Position der Elektrode darstellt, die durch die Elektrodenpositionsdaten angegeben wird.

8. Medizinische Vorrichtung nach einem der Ansprüche 1 bis 7, ferner umfassend eine externe Vorrichtung (140),
wobei ein Abschnitt der Verarbeitungsschaltung in der externen Vorrichtung enthalten ist und wobei die externe Vorrichtung eine Bildvorrichtung einschließt, die konfiguriert ist, um die das Bild des betreffenden Herzens und des Geräteabschnitts darstellenden Bilddaten zu erzeugen, die von der Bildgebungsvorrichtung generiert werden, insbesondere wobei die Bildgebungsvorrichtung ein Sichtfeld definiert und wobei die Bildvorrichtung konfiguriert ist, um die Bilddaten zu erzeugen, wenn sich das Bild des betreffenden Herzens und des Geräteabschnitts innerhalb des Sichtfelds befinden.

9. Medizinisches System nach einem der Ansprüche 1 bis 8, wobei die Verarbeitungsschaltung konfiguriert ist zum:
Empfangen eines Signals, das ein elektrisches Signal der Elektrode angibt, und
Aktualisieren des Trainingssatzes des Machine-Learning-Algorithmus unter Verwendung des angebenden Signals und der Elektrodenpositionsdaten.

10. Medizinisches System nach einem der Ansprüche 1 bis 9, wobei die Verarbeitungsschaltung konfiguriert ist zum:
Bestimmen einer Abmessung des Geräteabschnitts basierend auf einer oder mehreren physischen Strukturen an dem Geräteabschnitt, die von der Verarbeitungsschaltung erkannt werden können, wenn der Geräteabschnitt innerhalb des betreffenden Herzens positioniert ist; und
Generieren eines Teils der Elektrodenpositionsdaten basierend auf der Abmessung.

11. Medizinisches System nach einem der Ansprüche 1 bis 10, ferner umfassend den Geräteabschnitt, wobei der Geräteabschnitt konfiguriert ist, um ein elektrisches Signal zu generieren, wenn die Elektrode des Geräteabschnitts in elektrischer Verbindung mit Geweben des betreffenden Herzens steht, wobei der Geräteabschnitt konfiguriert ist, um steuerbar zu sein, wenn sich der Geräteabschnitt innerhalb des betreffenden Herzens befindet.

12. Medizinisches System nach einem der Ansprüche 1 bis 6, ferner umfassend:
eine medizinische Vorrichtung, die konfiguriert ist, um eine elektrische Verbindung mit dem Oberkörper eines Patienten herzustellen, wobei die medizinische Vorrichtung konfiguriert ist, um ein Signal zu erzeugen, das einen physiologischen Parameter eines betreffenden Herzens des Patienten darstellt; und
ein Gerät einschließlich eines Geräteabschnitts, der eine Elektrode trägt, wobei das Gerät konfiguriert ist, um ein elektrisches Signal zu erzeugen, wenn die Elektrode in elektrischer Verbindung mit Geweben des betreffenden Herzens steht, wobei der Geräteabschnitt konfiguriert ist, um steuerbar zu sein, wenn sich der Geräteabschnitt innerhalb des betreffenden Herzens befindet;
eine visuelle Anzeige;
wobei die Verarbeitungsschaltung ferner konfiguriert ist zum:
Extrahieren von Merkmalen des betreffenden Herzens unter Verwendung der empfangenen Bilddaten und Erzeugen eines repräsentativen Bildes des betreffenden Herzens auf der visuellen Anzeige unter Verwendung der extrahierten Merkmale;
Empfangen des repräsentatives Signals von der medizinischen Vorrichtung;

13. Medizinisches System nach Anspruch 12, wobei die Verarbeitungsschaltung konfiguriert ist zum:
Abbilden einer oder mehrerer Zielstellen des betreffenden Herzens unter Verwendung der empfangenen Bilddaten, wobei die eine oder die mehreren Zielstellen eine Position an dem betreffenden Herzen beschreiben;
Ermitteln der Position der Elektrode innerhalb des betreffenden Herzens unter Verwendung der Elektrodenpositionsdaten;
Vergleichen der ermittelten Position der Elektrode mit der einen oder den mehreren Zielstellen und
Generieren der Ausgabe basierend auf dem Vergleich der ermittelten Position der Elektrode mit der einen oder den mehreren Zielstellen; und/oder
wobei die Verarbeitungsschaltung konfiguriert ist zum:
Empfangen eines Signals, das das elektrische Signal der Elektrode angibt, und
Basieren der Ausgabe auf dem Signal, das das elektrische Signal angibt.

## Revendications

1. Système médical (100) comprenant :
un dispositif d'imagerie (130) configuré pour générer une image représentant le cœur d'un sujet (22) d'un patient (16) et représentant une partie d'appareil (120) à l'intérieur du cœur du sujet dans lequel la partie d'appareil est au moins une partie d'un appareil (12), et dans lequel la partie d'appareil comporte une électrode (124) ; et
un circuit de traitement (132) configuré pour :
recevoir des données d'image représentant l'image du cœur d'un sujet et de la partie d'appareil à l'intérieur du cœur du sujet,
acquérir des données de position d'électrode indiquant une position de l'électrode à l'intérieur du cœur du sujet, et
appliquer un algorithme d'apprentissage automatique pour générer un résultat lisible par un clinicien indiquant une probabilité de réussite de la stimulation du système de conduction en fonction des données de position d'électrode,
dans lequel l'algorithme d'apprentissage automatique est entraîné à l'aide d'un ensemble de données d'entraînement indiquant une ou plusieurs positions d'une électrode implantée à l'intérieur d'un cœur anatomique lorsque l'électrode implantée a établi avec succès et sans succès la stimulation du système de conduction du cœur anatomique.

2. Système médical selon la revendication 1, dans lequel le circuit de traitement est configuré pour :
cartographier un ou plusieurs sites cibles (14, 15) du cœur du sujet à l'aide des données d'image reçues, dans lequel le ou les sites cibles décrivent un emplacement sur le cœur du sujet ;
évaluer la position de l'électrode à l'intérieur du cœur du sujet à l'aide des données de position d'électrode ;
comparer la position évaluée de l'électrode au ou aux sites cibles ; et
générer le résultat en fonction de la comparaison de la position évaluée de l'électrode et du ou des sites cibles.

3. Système médical selon la revendication 1 ou 2, dans lequel le circuit de traitement est configuré pour :
recevoir un signal indiquant un signal électrique détecté par l'intermédiaire de l'électrode, et
baser le résultat sur le signal indiquant le signal électrique.

4. Système médical selon l'une quelconque des revendications 1 à 3, comprenant en outre un dispositif médical (150) couplé de manière opérationnelle au circuit de traitement, dans lequel :
le dispositif médical est configuré pour établir une communication électrique avec un torse du patient (16),
le dispositif médical est configuré pour générer un signal représentant un paramètre physiologique du cœur du sujet, et
le circuit de traitement est configuré pour baser le résultat sur le signal représentant le paramètre physiologique du cœur du sujet,
en particulier dans lequel le dispositif médical comprend une ou plusieurs électrodes de dispositif configurées pour établir une communication électrique avec le torse du patient.

5. Système médical selon l'une quelconque des revendications 1 à 4, dans lequel le circuit de traitement est configuré pour extraire les caractéristiques du cœur du sujet à l'aide des données d'image et d'un algorithme de reconnaissance d'image, dans lequel l'algorithme de reconnaissance d'image est entraîné à l'aide de données d'image d'entraînement représentant un cœur physiologique distinct du cœur du sujet.

6. Système médical selon la revendication 5, dans lequel le circuit de traitement est configuré pour acquérir les données de position d'électrode à l'aide des caractéristiques extraites ; et/ou
dans lequel le circuit de traitement est configuré pour :
déterminer un niveau de confiance en fonction des caractéristiques extraites, dans lequel le niveau de confiance est en fonction d'au moins l'une parmi une fidélité visuelle des caractéristiques extraites ou une précision de la position des caractéristiques extraites ; et
fournir une instruction de procédure au résultat lisible par le clinicien afin d'améliorer le niveau de confiance.

7. Système médical selon l'une quelconque des revendications 1 à 6, comprenant en outre un affichage visuel (142),
dans lequel le circuit de traitement est configuré pour extraire les caractéristiques du cœur du sujet à l'aide des données d'image reçues et produire une image représentant le cœur du sujet sur l'affichage visuel à l'aide des caractéristiques extraites, en particulier dans lequel le circuit de traitement est configuré pour indiquer un emplacement d'électrode représentatif sur l'image représentative, dans lequel l'emplacement d'électrode représentatif représente la position de l'électrode indiquée par les données d'emplacement d'électrode.

8. Système médical selon l'une quelconque des revendications 1 à 7, comprenant en outre un dispositif externe (140),
dans lequel une partie du circuit de traitement est contenue dans le dispositif externe, et dans lequel le dispositif externe comporte un dispositif d'image configuré pour produire les données d'image représentant l'image du cœur du sujet et la partie d'appareil générée par le dispositif d'imagerie, en particulier dans lequel le dispositif d'image définit un champ de vision, et dans lequel le dispositif d'image est configuré pour produire les données d'image lorsque l'image du cœur du sujet et la partie d'appareil se trouvent dans le champ de vision.

9. Système médical selon l'une quelconque des revendications 1 à 8, dans lequel le circuit de traitement est configuré pour
recevoir un signal indiquant un signal électrique de l'électrode, et
mettre à jour l'ensemble d'entraînement de l'algorithme d'apprentissage automatique à l'aide du signal indicatif et des données de position d'électrode.

10. Système médical selon l'une quelconque des revendications 1 à 9, dans lequel le circuit de traitement est configuré pour :
déterminer une dimension de la partie d'appareil en fonction d'une ou plusieurs structures physiques sur la partie d'appareil reconnaissable par le circuit de traitement lorsque la partie d'appareil est positionnée à l'intérieur du cœur du sujet ; et
générer une partie des données de position d'électrode en fonction de la dimension.

11. Système médical selon l'une quelconque des revendications 1 à 10 comprenant en outre la partie d'appareil, dans lequel la partie d'appareil est configurée pour générer un signal électrique lorsque l'électrode de la partie d'appareil est en communication électrique avec les tissus du cœur du sujet, dans lequel la partie d'appareil est configurée pour être orientable lorsque la partie d'appareil se trouve à l'intérieur du cœur du sujet.

12. Système médical selon l'une quelconque des revendications 1 à 6, comprenant en outre :
un dispositif médical configuré pour établir une communication électrique avec un torse d'un patient, dans lequel le dispositif médical est configuré pour générer un signal représentant un paramètre physiologique d'un cœur du sujet du patient ; et
un appareil comportant une partie d'appareil supportant une électrode, dans lequel l'appareil est configuré pour générer un signal électrique lorsque l'électrode est en communication électrique avec les tissus du cœur du sujet, dans lequel la partie d'appareil est configurée pour être orientable lorsque la partie d'appareil se trouve à l'intérieur du cœur du sujet ;
un affichage visuel ;
dans lequel le circuit de traitement est en outre configuré pour :
extraire les caractéristiques du cœur du sujet à l'aide des données d'image reçues et produire une image représentative du cœur du sujet sur l'affichage visuel à l'aide des caractéristiques extraites ;
recevoir le signal représentatif à partir du dispositif médical ;

13. Système médical selon la revendication 12, dans lequel le circuit de traitement est configuré pour :
cartographier un ou plusieurs sites cibles du cœur du sujet à l'aide des données d'image reçues, dans lequel le ou les sites cibles décrivent un emplacement sur le cœur du sujet ;
évaluer la position de l'électrode à l'intérieur du cœur du sujet à l'aide des données de position d'électrode ;
comparer la position évaluée de l'électrode au ou aux sites cibles ; et
générer le résultat en fonction de la comparaison de la position évaluée de l'électrode et du ou des sites ; et/ou.
dans lequel le circuit de traitement est configuré pour :
recevoir un signal indiquant le signal électrique de l'électrode, et
baser le résultat sur le signal indiquant le signal électrique.
